# EUROPEAN PATENT SPECIFICATION

(11) **EP 4 188 466 B1**
(45) Date of publication and mention of the grant of the patent: **25.12.2024**
(21) Application number: 21752666.4
(22) Date of filing: 27.07.2021
(51) Int. Cl.: A61L 24/00, A61L 31/12

(54) **BONE-ADHESIVE SHEET**
POLYOXAZOLINCOPOLYMER MIT CALCIUMBINDENDEN GRUPPEN
COPOLYMÈRE DE POLYOXAZOLINE COMPRENANT DES GROUPES DE LIAISON DE CALCIUM

(30) Priority: 28.07.2020 WO PCT/EP2020/071317; 06.11.2020 EP 20206146
(43) Date of publication of application: 07.06.2023
(73) Proprietor: Cilag GmbH International, 6300 Zug (CH)
(72) Inventor: LEEUWENBURGH, Sander Cornelis Gerardus, 6525 ED Nijmegen (NL); VAN HEST, Jan Cornelis Maria, 6525 ED Nijmegen (NL); SÁNCHEZ-FERNÁNDEZ, María José, 6525 ED Nijmegen (NL); LANAO, Rosa Pilar Felix, 6525 ED Nijmegen (NL); OPSTEEN, Joost, 6525 ED Nijmegen (NL); BENDER, Johannes Caspar Mathias Elizabeth, 6525 ED Nijmegen (NL)
(74) Representative: Nederlandsch Octrooibureau
(86) International application number: PCT/EP2021/071062
(87) International publication number: WO 2022/023375

(56) References cited:
- WO-A1-2013/137736
- WO-A1-2016/056901
- WO-A2-2010/059280
- US-A1- 2013 149 355
- US-A1- 2015 328 330
- US-B1- 6 733 845
- MISIC ANA M. ET AL: "Complete Genome Sequence and Methylome of Staphylococcus schleiferi , an Important Cause of Skin and Ear Infections in Veterinary Medicine", vol. 3, no. 5, 10 September 2015 (2015-09-10), XP055793878, Retrieved from the Internet <URL:https://www.ncbi.nlm.nih.gov/pmc/articles/PMC4566178/pdf/e01011-15.pdf> DOI: 10.1128/genomeA.01011-15
- ZHANG S ET AL: "The interaction of cationic polymers and their bisphosphonate derivatives with hydroxyapatite", MACROMOLECULAR BIOSCIENCE, WILEY-VCH VERLAG GMBH, DE, vol. 7, no. 5, 10 May 2007 (2007-05-10), pages 656 - 670, XP002546941, ISSN: 1616-5187, DOI: 10.1002/MABI.200600286

## Description

### TECHNICAL FIELD OF THE INVENTION

The present invention relates to a biocompatible, flexible, bone-adhesive sheet comprising:
- a cohesive fibrous carrier structure comprising a three-dimensional interconnected interstitial space;
- distributed within the interstitial space, (i) a plurality of polymer particles comprising a water-soluble calcium-binding polymer selected from polyoxazolines, polyethylene glycols, polyvinylpyrrolidones, polyurethanes, and combinations thereof, said water-soluble calcium-binding polymer carrying at least one calcium-binding group selected from bisphosphonate, citrate, ethylenediaminetetraacetic acid (EDTA) and combinations thereof or (ii) a plurality of reactive particles comprising an electrophilically activated water-soluble polymer selected from polyoxazolines, polyethylene glycols, polyvinylpyrrolidones, polyurethanes and combinations thereof and a plurality of bisphosphonate particles comprising nitrogenous bisphosphonate, wherein calcium-binding group is a group that is capable of forming two or more separate coordinate bonds with Ca²⁺ions.

The bone-adhesive sheet of the present invention is particularly suited for treatment of bone defects, e.g. by preventing fast-regenerating soft tissues from growing into more slowly regenerating bone tissue.

The invention further provides a method of preparing a bone-adhesive sheet, such as a barrier membrane, said method comprising:
- providing a water-resistant cohesive fibrous carrier structure comprising a three-dimensional interconnected interstitial space;
- providing polymer particles comprising a water-soluble calcium-binding polymer, said water-soluble calcium-binding polymer carrying at least one calcium-binding group or providing reactive particles comprising an electrophilically activated water-soluble polymer and bisphosphonate particles comprising nitrogenous bisphosphonate;
- impregnating the fibrous carrier structure with the polymer particles or impregnating the fibrous carrier structure with both the reactive particles and the bisphosphonate particles.

The present method enables effective distribution of the polymer particles inside the fibrous carrier structure and the production of bone-adhesive sheets with excellent adhesive properties.

### BACKGROUND OF THE INVENTION

Barrier membranes are routinely applied in surgery to allow for regeneration of alveolar bone, while various fixation devices are used in general and trauma surgery to fixate soft tissue to bone (tendon rupture), bone tissue to bone (fractures) or foreign implants to bone tissue (hernia repair with meshes). The current generation of commercially available, degradable barrier membranes and fixation devices has serious shortcomings related to i) a poor control over degradation rates, ii) poor mechanical properties, iii) poor understanding of biological mechanisms governing soft/bone tissue reconstruction and infection, iv) poor clinical manageability and stability, and v) long-term dependence of rigid non-degradable materials.

Currently, no biomaterials are available which adhere specifically to bone. Consequently, the development of medical devices which adhere specifically to bone would solve one of the major problems in oral, general and trauma surgery, namely the facile repair of damaged bone and/or fixation of soft or hard tissues to bone.

US 2013/0149355 describes a method to prevent protein and cell adsorption, said method comprising:
a) providing a water-soluble polymer comprising at one terminus, a hydroxyapatite-targeting moiety;
b) binding the water-soluble polymer from step (a) with a hydroxyapatite- or calcium phosphate-coated prosthetic or drug delivery device or surface under conditions effective to promote said binding to form a polymeric structure; and
c) administering the polymeric structure to a bone-containing mammal.

The hydroxyapatite-targeting moiety may be selected from the group consisting of tetracycline, calcein, bisphosphonates, polyaspartic acid, polyglutamic acid, and aminophosphosugars. The water-soluble polymer may be selected from the group consisting of poly(alkyleneglycol), poly(oxyethylated polyol), poly(olefinic alcohol), poly(vinylpyrolidone), poly(hydroxypropylmethacrylamide poly(α-hydroxy acid), poly(vinyl alcohol), polyphosphazene, polyoxazoline, and copolymers and terpolymers thereof.

Ossipov (Bisphosphonate-modified biomaterials for drug delivery and bone tissue engineering, Expert Opin Drug Deliv. (2015);12(9):1443-1458) reports that strategies of chemical immobilization of bisphosphonates in hydrogels and nanocomposites for bone tissue engineering have emerged that opened new applications of bisphosphonates in bone tissue engineering. Conjugates of bisphosphonates to different drug molecules, imaging agents, proteins and polymers are discussed in terms of specific targeting to bone and therapeutic effect induced. Conversion of these conjugates into hydrogel scaffolds is mentioned along with the application of the resulting materials for bone tissue engineering.

Zhang et al. (The interaction of cationic polymers and their bisphosphonate derivatives with hydroxyapatite, Macromol Biosci. (2007);7(5), 656-670) describe the construction of a polymeric linker containing multiple copies of BPs for protein conjugation and targeting to bone.

Poly(L-lysine) (PLL) and poly(ethylenimine) (PEI) were utilized as the polymeric backbones to incorporate a BP, namely 2-(3-mercaptopropylsulfanyl)-ethyl-1,1-bisphosphonic acid (thiolBP), by using *N-*hydroxysuccinimidyl polyethylene glycol maleimide and succinimidyl-4-(N-maleimidomethyl)-cyclohexane-1-carboxylate, respectively. In vitro and in vivo mineral affinity of the polymer-BP conjugates were determined in comparison with the unmodified polymers. The *in vitro* results indicated strong binding of the cationic polymers to HA in their unmodified form. BP conjugation did not enhance the inherent mineral affinity of the polymers; in contrast, certain modifications negatively affected the polymers' binding to the HA. In vivo results from a subcutaneous implant model in rats also showed no significant difference in mineral affinity of the BP modified and unmodified PE!.

Sánchez-Fernández et al. (Alendronate-functionalized poly(2-oxazoline)s with tunable affinity for calcium cations, Biomacromolecules (2019);20(8):2913-2921) reported the synthesis of a library of alendronate-functionalized polyoxazolines with control over the polymerization and functionalization degrees. The binding affinity of these polymers for calcium cations was much higher in comparison with other calcium-binding polymers reported previously. Results showed that adjusting the alendronate content in the polymer produced robust gels with a strong capacity for self-healing. The tunable synthetic versatility and affinity for calcium render these polymers excellent candidates for various applications in biomedicine.

WO 2010/059280 describes an anhydrous fibrous sheet comprising a first component of fibrous polymer, said polymer containing electrophilic groups or nucleophilic groups, and a second component capable of crosslinking the first component when said sheet is exposed to an aqueous medium in contact with biological tissue to form a crosslinked hydrogel that is adhesive to the biological tissue; wherein:
a) wherein the second component is a fibrous polymer having a backbone structure the same as or different from the fibrous polymer of the first component and containing electrophilic groups if the first component contains nucleophilic groups or containing nucleophilic groups if the first component contains electrophilic groups;
b) the second component is a coating on the fibrous polymer of the first component and wherein said coating contains electrophilic groups if the first component contains nucleophilic groups or nucleophilic groups if the first component contains electrophilic groups; or
c) the second component is a dry powder dispersed and entrapped within interstices of the fibrous polymer of the first component, wherein said powder contains electrophilic groups if the first component contains nucleophilic groups or nucleophilic groups if the first component contains electrophilic groups.

US 6,733,845 describes process for the electrostatic impregnation into a fibrous or filamentary network with powder, for producing a composite comprising a rigid or flexible matrix with which said network is in intimate contact, wherein the powder and said network of fibers or filaments are placed between two electrodes, said electrodes being electrically isolated insulated from each other and said electrodes being connected respectively to the oppositely charged poles of an AC voltage electrostatic generator so as to simultaneously subject said powder and said fibrous or filamentary network lying between said electrodes to an electrostatic field, the AC voltage of which is at least 5kV, for a time of at least 2 seconds.

### SUMMARY OF THE INVENTION

The inventors have developed a biocompatible, flexible, bone-adhesive sheet that is particularly suited for treatment of bone defects, e.g. by preventing fast-regenerating soft tissues from growing into more slowly regenerating bone tissue.

The bone-adhesive sheet according to the present invention comprises:
- a cohesive fibrous carrier structure comprising a three-dimensional interconnected interstitial space; and
- distributed within the interstitial space, (i) a plurality of polymer particles comprising a water-soluble calcium-binding polymer selected from polyoxazolines, polyethylene glycols, polyvinylpyrrolidones, polyurethanes and combinations thereof, said water-soluble calcium-binding polymer carrying at least one calcium-binding group selected from bisphosphonate, citrate, ethylenediaminetetraacetic acid (EDTA) and combinations thereof or (ii) a plurality of reactive particles comprising an electrophilically activated water-soluble polymer selected from polyoxazolines, polyethylene glycols, polyvinylpyrrolidones, polyurethanes and combinations thereof and a plurality of bisphosphonate particles comprising nitrogenous bisphosphonate,
wherein calcium-binding group is a group that is capable of forming two or more separate coordinate bonds with Ca²⁺ ions.

The bone-adhesive sheet of the present invention comprises a cohesive fibrous carrier structure that absorbs fluids and that can easily be impregnated with polymer particles. Unlike impregnation with liquids, such dry impregnation does not affect the structural integrity or mechanical properties of the carrier structure.

When aqueous liquid is absorbed by the bone-adhesive sheet of the present invention, the polymer particles within the sheet start dissolving as soon as they are 'wetted', thereby releasing the water-soluble polymer carrying calcium-binding groups. In an alternative embodiment, the water-soluble polymer carrying calcium-binding groups is formed *in situ* upon wetting when the electrophilically activated water-soluble polymer in the reactive particles reacts with the nitrogenous bisphosphonate in the bisphosphonate particles.

The polymer carrying calcium-binding groups is capable of forming a hydrogel due to reversible cross-linking with Ca²⁺ ions. This hydrogel strongly binds to bone. Thus, when applied onto bone tissue, the bone-adhesive sheet of the present invention is rapidly glued to the bone tissue by the adhesive action of the water-soluble calcium-binding polymer.

The polymer particles or the combination of reactive particles and bisphosphonate particles may be distributed homogeneously throughout the bone-adhesive sheet, thereby minimising bending friction.

Due to its flexibility, the bone-adhesive sheet of the present invention can suitably be applied to irregularly shaped sites.

Another aspect of the present invention relates to a method of preparing the bone-adhesive sheet of the present invention, said method comprising:
- providing a fibrous carrier structure comprising a three-dimensional interconnected interstitial space;
- providing polymer particles comprising a water-soluble calcium-binding polymer, said water-soluble calcium-binding polymer carrying at least one calcium-binding group or providing reactive particles comprising an electrophilically activated water-soluble polymer and bisphosphonate particles comprising nitrogenous bisphosphonate;
- impregnating the fibrous carrier structure with the polymer particles or impregnating the fibrous carrier structure with both the reactive particles and the bisphosphonate particles.

### DETAILED DESCRIPTION OF THE INVENTION

A first aspect of the invention relates to a bone-adhesive sheet comprising:
- a cohesive fibrous carrier structure comprising a three-dimensional interconnected interstitial space; and
- distributed within the interstitial space, (i) a plurality of polymer particles comprising a water-soluble calcium-binding polymer selected from polyoxazolines, polyethylene glycols, polyvinylpyrrolidones, polyurethanes and combinations thereof, said water-soluble calcium-binding polymer carrying at least one calcium-binding group selected from bisphosphonate, citrate, ethylenediaminetetraacetic acid (EDTA) and combinations thereof or (ii) a plurality of reactive particles comprising an electrophilically activated water-soluble polymer selected from polyoxazolines, polyethylene glycols, polyvinylpyrrolidones, polyurethanes and combinations thereof and a plurality of bisphosphonate particles comprising nitrogenous bisphosphonate,
wherein calcium-binding group is a group that is capable of forming two or more separate coordinate bonds with Ca²⁺ ions.

The term "interstitial space" as used herein refers to the void ("empty") space within the fibrous carrier structure. The interstitial space within the fibrous carrier structure allows the introduction of polymer particles into the sheet. Also blood and other bodily fluids can enter the interstitial space, allowing the water-soluble calcium-binding polymer within the polymer particles to dissolve.

The "water-soluble calcium-binding polymer" that is employed in accordance with the present invention has a molecular weight of at least 1 kDa and a solubility in distilled water of 20°C of at least 50 g/L.

The term "bisphosphonate" as used herein refers to a substance comprising two phosphonate groups that are interlinked by a central carbon atom by phosphoether bonds. The central carbon atom can carry two side chains referred to as R₁ and R₂.

The term "nitrogenous bisphosphonate" as used herein refers to a bisphosphonate wherein R₁ and/or R₂ contains nitrogen as part of an amine group.

The term "electrophilically activated water-soluble polymer" as used herein refers to a water-soluble polymer that comprises electrophilic groups that are capable of reacting with the amine group of the nitrogenous bisphosphonate under the formation of a covalent bond.

The term "water absorption capacity" as used herein is a measure of the capability of the bone-adhesive sheet to absorb water. The water absorption capacity is determined by weighing a sample of the dry sheet (weight = W_{d}) followed by immersion of the sample into distilled water (37°C) for 45 minutes. Next, the sample is removed from the water and water clinging to the outside of the substrate is removed, following which the sample is weighed again (weight = W_{w}). The water absorption capacity = 100% × (W_{w}-W_{d})/W_{d}. The water absorption capacity is indicative of the porosity of the substrate as well as of its ability to swell in the presence of water.

The term "collagen" as used herein refers the main structural protein in the extracellular space of various connective tissues in animal bodies. Collagen forms a characteristic triple helix of three polypeptide chains. Depending upon the degree of mineralization, collagen tissues may be either rigid (bone) or compliant (tendon) or have a gradient from rigid to compliant (cartilage). Unless indicated otherwise, the term "collagen" also encompasses modified collagens other than gelatin.

The term "gelatin" as used herein refers to a mixture of peptides and proteins produced by partial hydrolysis of collagen extracted from the skin, bones, and connective tissues of animals such as domesticated cattle, chicken, pigs, and fish. During hydrolysis, the natural molecular bonds between individual collagen strands are broken down into a form that rearranges more easily.

The term "polyoxazoline" as used herein refers to a poly(N-acylalkylenimine) or a poly(aroylalkylenimine) and is further referred to as POx. An example of POx is poly(2-ethyl-2-oxazoline). The term "polyoxazoline" also encompasses POx copolymers.

The diameter distribution of the polymer particles, the reactive particles and the bisphosphonate particles may suitably be determined by means of laser diffraction using a Malvern Mastersizer 2000 in combination with the Stainless Steel Sample Dispersion Unit. The sample dispersion unit is filled with approx. 120 mL of cyclohexane, which is stabilized for 5 to 10 minutes at a stirring speed of 1800 rpm, followed by a background measurement (blanc measurement). The sample tube is shaken and turned horizontally for 20 times. Next, about 50 mg is dispersed in the sample dispersion unit containing the cyclohexane. After the sample is introduced in the dispersion unit, the sample is stirred for one and a half minute at 1800 rpm to ensure that all particles are properly dispersed, before carrying out the measurement. No ultrasonic treatment is performed on the dispersed particles. Mean particle size is expressed as D [4,3], the volume weighted mean diameter (ΣniDi⁴)/(ΣniDi³).

According to a particularly preferred embodiment, the fibrous carrier structure is water-resistant. Here the term "water-resistant" means that the fibrous carrier structure is not water soluble and does not disintegrate in water to form a colloidal dispersion, at neutral pH conditions (pH 7) and a temperature of 37°C.

According to a particularly preferred embodiment, the bone-adhesive sheet of the present invention is bio-degradable, meaning that the sheet, the particles contained therein and any other components of the bone-adhesive sheet are eventually degraded in the body. Biodegradation of the sheet and particles typically requires chemical decomposition (e.g. hydrolysis) of polymers contained therein. Complete bio-degradation of the bone-adhesive sheet by the human body is typically achieved in 1 to 16 weeks, more preferably in 4 to 14 weeks, most preferably in 8 to 12 weeks.

The bone-adhesive sheet of the present invention is preferably sterile.

The bone-adhesive sheet of the present invention typically has a mean thickness of 0.1-25 mm. More preferably, the mean thickness is in the range of 0.1-10 mm, most preferably in the range of 0.2-5 mm.

The dimensions of the bone-adhesive sheet preferably are such that the top and bottom of the sheet each have a surface area of at least 2 cm², more preferably of at least 5 cm² and most preferably of 5-50 cm². Typically, the sheet is rectangular in shape and has a length of 25-200 mm, and a width of 20-200 mm.

The bone-adhesive sheet preferably has a density of less than 2 g/cm³, more preferably of less than 1 g/cm³ and most preferably of 0.2-0.8 g/cm³.

The bone-adhesive sheet of the present invention preferably is essentially anhydrous. Typically, the bone-adhesive sheet has a water content of not more than 5 wt.%, more preferably of not more than 2 wt.% and most preferably of not more than 1 wt.%.

The water absorption capacity of the bone-adhesive sheet preferably is at least 50%, more preferably lies in the range of 100% to 800%, most preferably in the range of 200% to 500%.

The fibres in the fibrous carrier structure preferably have a mean diameter of 1-500 µm, more preferably of 2-300 µm and most preferably of 5-200 µm. The mean diameter of the fibres can suitably be determined using a microscope.

Typically, at least 50 wt.%, more preferably at least 80 wt.% of the fibres in the fibrous carrier structure have a diameter of 1-300 µm and a length of at least 1 mm.

Preferably, at least 50 wt.%, more preferably at least 80 wt.% of the fibres in the fibrous carrier structure have an aspect ratio (ratio of length to diameter) of at least 1000.

The fibrous carrier structure that is employed in accordance with the present invention preferably is a felt structure, a woven structure or a knitted structure. Most preferably, the fibrous carrier structure is a felt structure. Here the term "felt structure" refers to a structure that is produced by matting and pressing fibres together to form a cohesive material.

The fibrous carrier structure preferably comprises at least 50 wt.%, more preferably at least 80 wt.% and most preferably at least 90 wt.% fibres containing gelatin, collagen, cellulose, modified cellulose, carboxymethyldextran, PLGA, sodium hyaluronate/carboxy methylcellulose, polyvinyl alcohol, chitosan or a combination thereof.

In an embodiment of the invention, the fibrous carrier structure does not comprise oxidised regenerated cellulose.

Preferred fibrous carrier structures have an open pore structure with a permeability to air of at least 0.1 L/min × cm², more preferably of at least 0.5 L/min × cm². The air permeability is determined in accordance with EN ISO 9237:1995 (Textiles - Determination of the permeability of fabrics to air).

The fibres in the fibrous carrier structure can be produced by means of methods known in the art, such as electrospinning, electro-blown spinning and high speed rotary sprayer spinning. Production of fibrous carrier structure by means of high speed rotary sprayer spinning is described in US 2015/0010612. It is also possible to use commercially available haemostatic fibrous sheets as the fibrous carrier structure.

In a preferred embodiment, the bone-adhesive sheet comprises, distributed within the interstitial space, a plurality of polymer particles comprising a water-soluble calcium-binding polymer, said water-soluble calcium-binding polymer carrying at least one calcium-binding group, as disclosed in claim 1.

The polymer particles may be homogeneously distributed within the interstitial space of the fibrous carrier structure in the sense that the particle density is essentially the same throughout the sheet. The polymer particles may be unevenly distributed through the thickness of the sheet. For certain applications it may be advantageous if the polymer particle density shows a gradient, e.g. in that the density of polymer particles is lowest near the side of the sheet that is meant to applied onto a bleeding wound and highest near the other side of the sheet.

The bone-adhesive sheet of the present invention preferably comprises 5-90%, more preferably 10-80%, even more preferably 20-75% and most preferably 50-70% of the polymer particles, said percentage being calculated by weight of the fibrous carrier structure.

The polymer particles employed in the bone-adhesive sheet preferably contain at least 10 wt.% of the water-soluble calcium-binding polymer. More preferably, the polymer particles contain at least 50 wt.%, more preferably at least 90 wt.% of the water-soluble calcium-binding polymer.

In a very preferred embodiment, the polymer particles have a volume weighted mean diameter (D [4,3], (ΣniD ⁴)/(ΣniD ³)) in the range of 5-220 µm, more preferably 10-150 µm, even more preferably in the range of 15-120 µm and most preferably in the range of 20-100 µm.

According to a particularly preferred embodiment at least 80 vol.% of the polymer particles have a diameter in the range of 5-200 µm, more preferably in the range of 10-120 µm and most preferably in the range of 12-100 µm.

The polymer particles of the present invention may be prepared in various ways, e.g. by milling, by spray drying a polymeric solution, by freeze drying, by spray chilling a polymeric melt, by granulating a powder mixture, or by fluidised bed coating.

The water-soluble calcium-binding polymer that is contained in the polymer particles typically has a molecular weight of at least 2 kDa, more preferably of at least 5 kDa and most preferably of 10-100 kDa.

The water-soluble calcium-binding polymer preferably has a solubility in distilled water of 20°C of at least 100 g/L, more preferably of at least 200 g/L.

The affinity of the water-soluble calcium-binding polymer to hydroxyapatite is dependent on the number of calcium-binding groups in the copolymer. Preferably, the polymer contains at least 4 calcium-binding groups, more preferably at least 6 calcium-binding groups, even more preferably at least 8 calcium-binding groups and most preferably at least 10 calcium-binding groups. Typically, the polymer contains not more than 200 calcium-binding groups.

On average the water-soluble calcium-binding polymer carries 0.05-5 calcium-binding groups, more preferably 0.1-3 calcium-binding groups, most preferably 0.2-2 calcium-binding groups per kDa.

The water-soluble calcium-binding polymer that is present in the polymer particles is selected from polyoxazolines, polyethylene glycols, polyvinylpyrrolidones, polyurethanes (e.g. as described in WO 2017/171551) and combinations thereof. Even more preferably the calcium-binding polymer is selected from polyoxazolines, polyethylene glycols and combinations thereof. Most preferably the calcium-binding polymer is a polyoxazoline.

The polyoxazoline comprising calcium-binding groups is preferably derived from a polyoxazoline whose repeating units are represented by the following formula (I):

(CHR¹)ₘNCOR²

wherein R², and each of R¹ are independently selected from H, optionally substituted C₁₋₂₂ alkyl, optionally substituted cycloalkyl, optionally substituted aralkyl, optionally substituted aryl; and m being 2 or 3.

Preferably, R¹ and R² in formula (I) are selected from H and C₁₋₈ alkyl, even more preferably from H and C₁₋₄ alkyl. R¹ most preferably is H. The integer m in formula (I) is preferably equal to 2.

According to a preferred embodiment, the polyoxazoline is a polymer, even more preferably a homopolymer of 2-alkyl-2-oxazoline, said 2-alkyl-2-oxazoline being selected from 2-methyl-2-oxazoline, 2-ethyl-2-oxazoline, 2-propyl-2-oxazoline, 2-butyl-2-oxazoline and combinations thereof. Preferably, the polyoxazoline is a homopolymer of 2-propyl-2-oxazoline or 2-ethyl-oxazoline. Most preferably, the polyoxazoline is a homopolymer of 2-ethyl-oxazoline.

According to a particularly preferred embodiment, the water-soluble calcium-binding polymer comprises at least 20 oxazoline units, more preferably at least 30 oxazoline units and most preferably at least 80 oxazoline units. The calcium-binding polymer preferably comprises on average at least 0.05 calcium-binding groups per oxazoline residue. Even more preferably, the calcium-binding polymer comprises on average at least 0.1 calcium-binding groups per oxazoline residue. Most preferably, the calcium-binding polymer comprises on average 0.12-0.5 calcium-binding groups per oxazoline residue.

Oxazoline units containing a calcium-binding group preferably represent 3-50% of the monomeric units contained in the water-soluble calcium-binding polymer More preferably, the calcium-binding group containing oxazoline units represent 4-40%, most preferably 5-35% of the monomeric units contained in the water-soluble calcium-binding polymer.

According to a particularly preferred embodiment, the water-soluble calcium-binding polymer comprises:
repeating units A of formula -[N(R^{a})CH₂CH₂]-; and
repeating units B of formula -[N(R^{b})CH₂CH₂]-;
wherein:
   R^{a} is CO-(CHR¹)ₜ-H
   R^{b} is CO-(CHR¹)ₜ-CONH-R²-X-CO-(CHR³)ᵤ-CONR⁴-CO-*ccr*
   R¹ represents H or optionally substituted C₁₋₅ alkyl;
   R² represents an optionally substituted C₁₋₅ alkylene;
   R³ represents H or optionally substituted C₁₋₅ alkyl;
   R⁴ represents H or CH₃;
   X represents O, NR¹¹, S or ⁺NR¹¹(R¹²);
   R¹¹ and R¹² represent H, methyl or ethyl;
   t represents 1, 2 or 3;
   u represents 1, 2 or 3;
   *ccr* represents a calcium-binding group.

The calcium-binding group (ccr in the formula representing repeating units B) represents a group selected from bisphosphonate, citrate, ethylenediaminetetraacetic acid (EDTA) and combinations thereof. More preferably, the calcium-binding group comprises bisphosphonate. Even more preferably, the calcium-binding group (ccr) represents a group comprising bisphosphonate that is represented by formula (I):

-(L)C(Z)(PO₃H₂)₂ (I)

or a pharmaceutically acceptable salt thereof;
wherein:
   L represents an optionally substituted C₁₋₅ alkylene;
   Z represents H, OH, CI, F or a methyl group.

In the above mentioned formula (I), L preferably represents C₂₋₄ alkylene. Most preferably, L represents propylene.

In formula (I), Z preferably represents H, OH or a methyl group. Most preferably, Z represents OH.

According to a preferred embodiment, R¹ in the abovementioned formulae of the repeating units A and B represents H.

The integer t in the formulae of the repeating units A and B preferably is 2 or 3.

In the formulae of the repeating units B, R² preferably represents ethylene.

The integer u in the formula of the repeating unit B preferably is 2 or 3.

R³ in the formula of repeating unit B preferably represents H.

R⁴ in the formula of repeating unit B preferably represents H.

In the formulae of repeating units B, X preferably represents O.

According to preferred embodiment, the water-soluble calcium-binding polymer of the present invention has the following monomeric composition comprises 40-97 mol.% of repeating units A and 3-50 mol.% of repeating units B. Even more preferably, the water-soluble calcium-binding polymer comprises 50-95 mol.% of repeating units A and 4-40 mol.% of repeating units B.

Most preferably, the water-soluble calcium-binding polymer comprises 55-92 mol.% of repeating units A and 5-35 mol.% of repeating units B.

Together, the repeating units A and B preferably constitute at least 55%, more preferably at least 65% and most preferably at least 70% of the monomeric units present in the water-soluble calcium-binding polymer.

Another aspect, not part of the present invention, relates to the use of the water-soluble calcium-binding polymer of the present invention for imparting bone adhesiveness to a medical product.

Polyoxazoline can carry calcium-binding groups in its side chains (pendant calcium-binding groups), at its termini, or both. The polyoxazoline that is employed in accordance with the present invention advantageously contains one or more pendant calcium-binding groups. Typically, the polyoxazoline contains 0.03-0.5 pendant calcium-binding groups per monomer, more preferably 0.04-0.35 pendant calcium-binding groups per monomer, even more preferably 0.05-0.25 pendant calcium-binding groups per monomer.

The polyethylene glycol (PEG) comprising calcium-binding groups that is applied in accordance with the present invention preferably is a multi-arm PEG comprising at least 3 arms, more preferably at least 4 arms, most preferably at least 6 arms. Typically, the multi-arm PEG does not contain more than 20 arms.

Preferably, the multi-arm PEG comprises at least 3 arms that are terminated with one or two calcium-binding groups. Most preferably each arm of the multi-arm PEG is terminated with one or two calcium-binding groups.

An example of a multi-armed PEG having 4 arms each terminated with one calcium-binding group R and an example of a multi-armed PEG comprising 8 arms each terminated with one calcium-binding groups R are shown in Figure 1.

In accordance with another preferred embodiment, the bone-adhesive sheet comprises, distributed within the interstitial space, a plurality of reactive particles comprising an electrophilically activated water-soluble polymer and a plurality of bisphosphonate particles comprising nitrogenous bisphosphonate, as disclosed in claim 1.

The reactive particles and the bisphosphonate particles may be separately distributed within the interstitial space. According to a particularly preferred embodiment, however, the reactive particles and the bisphosphonate particles are combined in agglomerates and these agglomerates are distributed within the interstitial space. Combining the reactive particles and the bisphosphonate particles in agglomerates offers the advantage that the *in situ* formation of water-soluble calcium-binding polymer proceeds very rapidly when the bone-adhesive sheet absorbs aqueous liquid.

The agglomerates of reactive particles and bisphosphonate particles preferably have a volume weighted mean diameter (D [4,3], (ΣniD ⁴)/(ΣniD ³)) in the range of 100-400 µm, more preferably in the range of 200-350 µm and most preferably 220-320- µm.

According to a preferred embodiment at least 80 vol.% of the agglomerates has a diameter in the range of 150-350 µm, more preferably in the range of 200-300 µm and most preferably in the range of 225-275 µm.

The bone-adhesive sheet of the present invention preferably comprises 10-140%, more preferably 20-130%, even more preferably 40-120% and most preferably 50-110% of the agglomerates of reactive particles, said percentage being calculated by weight of the fibrous carrier structure.

The bone-adhesive sheet of the present invention preferably comprises 1.6-37%, more preferably 3.2-34%, even more preferably 6.4-32% and most preferably 8.3-29% of the bisphosphonate particles, said percentage being calculated by weight of the fibrous carrier structure.

The reactive particles employed in the bone-adhesive sheet preferably contain at least 10 wt.% of the electrophilically activated water-soluble polymer. More preferably, the reactive particles contain at least 50 wt.%, more preferably at least 90 wt.% of the electrophilically activated water-soluble polymer.

The bisphosphonate particles employed in the bone-adhesive sheet preferably contain at least 10 wt.% of the nitrogenous bisphosphonate. More preferably, the reactive particles contain at least 50 wt.%, more preferably at least 90 wt.% of the nitrogenous bisphosphonate.

The nitrogenous bisphosphonate in the bisphosphonate particles is preferably selected from alendronate, pamidronate, neridronate, olpadronate, ibandronate and combinations thereof. More preferably, the nitrogenous bisphosphonate is selected from alendronate, pamidronate, neridronate and combinations thereof.

The electrophilically activated water-soluble polymer that is contained in the reactive particles preferably comprises one or more electrophilic groups selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato (isothiocyanato), isocyano, epoxides, activated hydroxyl groups, olefins, glycidyl ethers, carboxyl, succinimidyl esters, succinimidyl carbonates, succinimidyl carbamates, sulfosuccinimidyl esters, sulfosuccinimidyl carbonates, maleimido (maleimidyl), ethenesulfonyl, imido esters, aceto acetate, halo acetal, orthopyridyl disulfide, dihydroxyphenyl derivatives, vinyl, acrylate, acrylamide, iodoacetamide and combinations thereof. More preferably, the electrophilic groups are selected from: carboxylic acid esters, acid chloride groups, anhydrides, ketones, aldehydes, isocyanato, thioisocyanato, epoxides, activated hydroxyl groups, olefins, carboxyl, succinimidyl ester, succinimidyl carbonate, succinimidyl carbamates, sulfosuccinimidyl ester, sulfosuccinimidyl carbonate, maleimido, ethenesulfonyl and combinations thereof. Even more preferably, the electrophilic groups are selected from aldehydes, isocyanato, thioisocyanato, succinimidyl ester, sulfosuccinimidyl ester, maleimido and combinations thereof. Most preferably, the electrophilic groups are selected from isocyanato, thioisocyanato, succinimidyl ester, sulfosuccinimidyl ester, maleimido and combinations thereof.

The electrophilically activated water-soluble polymer employed in the present method preferably contains on average at least 6, more preferably at least 12 and most preferably 15 to 30 electrophilic groups.

The electrophilically activated water-soluble polymer preferably contains on average at least 0.1 electrophilic groups per monomer, more preferably 0.12 to 2 electrophilic groups per monomer, most preferably 0.15 to 0.6 electrophilic groups per monomer.

The electrophilically activated water-soluble polymer that is contained in the reactive particles typically has a molecular weight of at least 2 kDa, more preferably of at least 5 kDa and most preferably of 10-100 kDa.

The electrophilicaly water-soluble polymer preferably has a solubility in distilled water of 20°C of at least 100 g/L, more preferably of at least 200 g/L.

The electrophilically activated water-soluble polymer is selected from polyoxazolines, polyethylene glycols, polyvinylpyrrolidones, polyurethanes (e.g. as described in WO 2017/171551) and combinations thereof. Even more preferably the electrophilically activated water-soluble polymer is selected from polyoxazolines, polyethylene glycols and combinations thereof. Most preferably electrophilically activated water-soluble polymer is a polyoxazoline.

The electrophilically activated polyoxazoline is preferably derived from a polyoxazoline as defined herein before.

According to another preferred embodiment, the bone adhesive sheet comprises an occlusive backing layer. Preferably, this backing layer is water-resistant. Here the term "water-resistant" means that the backing layer is not water soluble and does not disintegrate in water to form a colloidal dispersion, at neutral pH conditions (pH 7) and a temperature of 37°C.

The backing layer typically has an average thickness of 5-100 µm, more preferably an average thickness of 10-80 µm and most preferably of 20-60 µm.

The backing layer preferably contains at least 50 wt.% of one or more polymers selected from poly(L-lactide-co-caprolactone) (PLC), poly(D,L-lactic-co-glycolic acid) (PLGA), and poly(2-propyl-2-oxazoline) functionalized with pendant amine groups (P(PropOx-NH₂).

Another aspect of the present invention relates to a method of preparing the bone-adhesive sheet as described herein before, said method comprising:
- providing a fibrous carrier structure comprising a three-dimensional interconnected interstitial space;
- providing polymer particles comprising a water-soluble calcium-binding polymer, said water-soluble calcium-binding polymer carrying at least 3 calcium-binding groups or providing reactive particles comprising an electrophilically activated water-soluble polymer and bisphosphonate particles comprising nitrogenous bisphosphonate;
- impregnating the fibrous carrier structure with the polymer particles or impregnating the fibrous carrier structure with both the reactive particles and the bisphosphonate particles.

The fibrous carrier structure employed in the present method preferably is a fibrous carrier structure as described herein before. Likewise, the polymer particles, the reactive particles, the bisphosphonate particles, the water-soluble calcium-binding polymer, the electrophilically activated water-soluble polymer and the nitrogenous bisphosphonate preferably are as described herein before.

The reactive particles and bisphosphonate particles are preferably provided together in the form of an agglomerate containing both reactive particles and bisphosphonate particles.

According to a particularly preferred embodiment of the present method, the fibrous carrier structure is impregnated by:
- placing the fibrous carrier structure and the polymer particles between two electrodes;
- simultaneously subjecting the fibrous carrier structure and the polymer particles to an electric field of 0.1 to 40 kV/mm to impregnate the interconnected interstitial space of the fibrous carrier structure with the polymer particles.

According to an alternative preferred embodiment of the present method, the fibrous carrier structure is impregnated by:
- placing the fibrous carrier structure, reactive particles and the bisphosphonate particles between two electrodes;
- simultaneously subjecting the fibrous carrier structure, the reactive particles and the bisphosphonate particles to an electric field of 0.1 to 40 kV/mm to impregnate the interconnected interstitial space of the fibrous carrier structure with the reactive particles and bisphosphonate particles.

The use of an electric field enables deep impregnation with, and a homogeneous distribution of the particles within the fibrous carrier structure. In addition, the present method offers the advantage that, unlike impregnation with liquids, it does not affect the structural integrity or mechanical properties of the fibrous sheet. Furthermore, in comparison to mechanical impregnation methods that make use of shaking or vibration, the present method does not impose mechanical stress and achieves a more effective impregnation, especially with very small (<100 µm) particles.

In the present method the fibrous carrier structure and the particles are preferably simultaneously subjected to an electric field of 0.5 to 30 kV/mm, more preferably to an electric field of 1 to 10 kV/mm.

According to a particularly preferred embodiment, the fibrous carrier structure and the particles are simultaneously subjected to an alternating electric field. Preferably, the electric field is alternated with a frequency of at least 10 s⁻¹, more preferably with a frequency of at least 50 s⁻¹ and most preferably with a frequency of 100 s⁻¹.

Impregnation of the fibrous carrier structure may effectively be achieved in the present method by creating a layer of the particles adjacent to the fibrous carrier structure and by placing the fibrous carrier structure and the adjacent layer of particles between the two electrodes. Impregnation may also be achieved by creating a laminate containing two or more fibrous carrier structures separated by layers of particles and by placing this laminate between the two electrodes.

In a preferred embodiment of the present method, the fibrous carrier structure and the particles are placed between a lower electrode and an upper electrode, these electrodes being electrically insulated from each other by a dielectric and connected to the respective poles of an AC generator so as to simultaneously subject the fibrous carrier structure and the particles to the electric field.

The fibrous carrier structure and the particles are preferably passed between the lower electrode and upper electrode whilst simultaneously subjecting the fibrous carrier structure and the particles to the electric field. Thus, a roll of fibrous carrier structure may suitably be impregnated with the particles by the present method in a semi-continuous fashion.

In a preferred embodiment, the fibrous carrier structure and the particles are simultaneously exposed to the electric field for at least 0.1 second, more preferably for at least 5 seconds and most preferably for at least 30 seconds.

The invention is further illustrated by the following non-limiting examples.

### EXAMPLES

### Materials

- 2-ethyl-2-oxazoline (EtOx) was obtained from Sigma-Aldrich.
- 2-methoxycarbonylethyl-2-oxazoline (MestOx) was synthesised as described by Bouten et al. (Accelerated living cationic ring-opening polymerization of a methyl ester functionalized 2-oxazoline monomer, Polym. Chem. (2015), 6, 514-518).
- Acetonitrile and tetrahydrofuran were discharged under nitrogen atmosphere using an MBraun MB SPS-800 solvent dispersing system.
- Anhydrous dichloromethane (DCM), anhydrous diethyl ether, HPLC grade methanol, and fused calcium chloride were obtained from Fisher Scientific.
- Anhydrous *N*,*N*-dimethylformamide (DMF) was obtained from Acros Organics.
- Succinic anhydride was obtained from Alfa Aesar.
- *N*-(3-dimethylaminopropyl)-*N'*-ethylcarbodiimide hydrochloride (EDC), 4-dimethylamino pyridine (DMAP), *N*,*N'*-diisopropylcarbodiimide (DIC), *N*-hydroxysuccinimide (NHS), and sodium alendronate trihydrate (Ale) were obtained from Fluorochem.
- Alendronic acid was obtained from TCI.
- Dimethyl sulfoxide, 2-ethanolamine, methyl-p-toluenesulfonate (MeOTs), and α-cyano-4-hydroxycinnamic acid (CHCA) were obtained from Sigma-Aldrich.
- Ultrapure Milli-Q water set to 18.2 MΩ/cm was obtained from a WaterPro PS polisher (Labconco, Kansas City, MO).
- Dialysis membranes Spectra/Por^{®} 3 (3.5 kD cutoff) were obtained from VWR International.
- Phosphate buffered saline (PBS) solution was prepared containing 2.7 mM KCI, 137 mM NaCl, 2 mM KH₂PO₄, and 8 mM Na₂HPO₄ (pH = 7.4).
- Poly(2-ethyl-2-oxazoline) average Mw -50,000, PDI 3-4 was obtained from Sigma-Aldrich.
- Hydroxyapatite (Calcium Phosphate, CaP) was obtained from Merck.
- 8-Arm poly(ethylene glycol) succinimidyl *N*-hydroxysuccinimide ester Mw ~ 10,000 was obtained from Creative PEGWorks.

### Synthesis of alendronate-functionalized poly(2-oxazoline)s.

Alendronate-functionalized poly(2-oxazoline)s were synthesized in five steps as shown in Figure 2.

2-Methoxycarbonylethyl-2-oxazoline (MestOx) was copolymerized either with 2-ethyl-2-oxazoline (EtOx) to yield a randomly distributed statistical copolymer. Methyl-p-toluenesulfonate (1 equiv), EtOx (m equiv), MestOx (n equiv), and dry acetonitrile (4 M) were mixed under inert atmosphere in the desired ratios in microwave vials. The polymerization was carried out under microwave irradiation at 140 °C for 15 min following a cationic ring opening mechanism (CROP). After polymerization, the reaction was terminated by the addition of ethanolamine (10 equiv) stirring for 30 min at room temperature. Then, the solvent was evaporated *in vacuo* to afford P(EtOx-ran-MestOx) copolymer **P1a.**

The copolymer **P1a** (1 equiv) was modified by direct amidation with 2-ethanolamine (3.5 equiv) at 60 °C under reduced pressure (30 kPa) (300 mbar) for 16 h. Then, the crude mixtures were purified by three consecutive precipitations in a mixture of acetone:ether v/v 3:1 and re-dissolved in DCM:MeOH v/v 8:2, followed by ion-exchange chromatography in MeOH. Finally, the solvents were evaporated under vacuum to afford P(EtOx-OH) copolymer **P1b.**

The hydroxyl side-functionalized polymer **P1b** (1 equiv) was either fully or partially converted to carboxylic acid moieties using succinic anhydride (1.1 equiv), 4-dimethylamino pyridine (DMAP) (1.1 equiv), and dissolved in DCM:DMF v/v 9:1 (2 M) under argon atmosphere for 16 h. The crude mixtures were purified by three consecutive precipitations in acetone and re-dissolved in DCM:MeOH v/v 8:2, followed by ion-exchange chromatography in MeOH. Finally, the solvent was evaporated under vacuum to afford either P(EtOx-COOH) or P(EtOx-OH-COOH) **P1c** and **P2c.**

These carboxylate side-functionalized copolymers were subsequently modified into reactive esters by carbodiimide coupling with *N*-hydroxysuccinimide. The functionalized polymers were dissolved in DCM:DMF v/v 95:5 (0.2 M), *N*-hydroxysuccinimide (1.1 equiv) and *N,N'-*diisopropylcarbodiimide (1.2 equiv) as coupling agent were added, and stirred under argon atmosphere at room temperature for 16 h. They were purified by two precipitations in acetone:ether v/v 1:1 followed by other in ether, and re-dissolved in DCM. Finally, the solvents were evaporated under vacuum to afford P(EtOx-OH-NHS) or P(EtOx-NHS) **P1d and P2d.** Alendronate moieties were incorporated in the polymer side chain by an amidation reaction. The NHS-activated copolymers **P1d** and **P2d** (1 equiv) were slowly added into a solution containing sodium alendronate trihydrate (2 equiv), *N*-hydroxysuccinimide (1 equiv) and *N-*(3-dimethylaminopropyl)-*N'*-ethylcarbodiimide hydrochloride (1 equiv) in phosphate buffered saline (0.5 M), stirring at 3 °C for 4 h with the pH previously adjusted to 7.7 using NaOH. Afterwards, the mixtures were dialyzed for 16 h, follow by three precipitations in acetone:ether v/v 3:1, and re-dissolved in demi-water. Finally, they were lyophilized to afford either P(EtOx-Ale), P(EtOx-OH-Ale) copolymers **P1e** and **P2e.**

### Example 1

### Synthesis of P(EtOx₇₀-Ale₃₀) - P1e

### P(EtOx₈₀-MestOx₂₀) P1a

According to the general procedure, the reaction of a solution of methyl tosylate (0.46 mL, 3.01 mmol), 2-ethyl-2-oxazoline (24.32 mL, 240.94 mmol) and 2-methoxycarbonylethyl-2-oxazoline (8.31 mL, 60.24 mmol) in dry acetonitrile (43 mL, 4 M), terminated by the addition of 2-ethanolamine (1.82 mL, 30.12 mmol) afforded the desired polymer **P1a** (36.22 g, 2.93 mmol). **¹H NMR** [400 MHz, δ (ppm), CDCl₃]: 3.65 (br, 3 H, 5-C*H*₃), 3.65-3.35 (br, 8 H, 1-C*H*₂), 2.75-2.50 (br, 4 H, 4-C*H*₂), 2.50-2.20 (br, 2 H, 2-C*H*₂), 0.95-1.15 (br, 3 H, 3-C*H*₃). Experimentally determined copolymer ratio: m/n 80:20. **SEC:** Mₙ 9.6 kDa, Ð: 1.17. **MALDI-TOF:** Mₙ 9.8 kDa. **Yield:** 97%.

### P(EtOX₇₀-OH₃₀) P1b

According to the general procedure, the reaction of **P1a** (51 g, 4.35 mmol) with 2-aminoethanol (27.55 mL, 456.55 mmol) afforded the desired polymer **P1b** (43.98 g, 3.49 mmol). **¹H NMR** [400 MHz, δ (ppm), D₂O]: 3.66 (br, 2 H, 7-C*H*₂), 3.75-3.45 (br, 8 H, 1-C*H*₂), 3.35-3.25 (br, 2 H, 6-C*H*₂), 2.75-2.60 (br, 2 H, 5-C*H*₂), 2.60-2.50 (br, 2 H, 4-C*H*₂), 2.45-2.25 (br, 2 H, 2-C*H*₂), 1.00-0.80 (br, 3 H, 3-C*H*₃). Experimentally determined copolymer ratio: m/n 70:30. MALDI-**TOF:** Mₙ 11.1 kDa. **Yield:** 80%.

### P(EtOx₇₀-COOH₃₀) P1c

According to the general procedure, the reaction of a solution of **P1b** (10 g, 0.79 mmol), 4-dimethylamino pyridine (0.58 g, 4.76 mmol) and succinic anhydride (2.86 g, 28.54 mmol) in DCM/ACN 9:1 (17 mL, 2 M) afforded the desired polymer **P1c** (9.85 g, 0.64 mmol). **¹H NMR** [400 MHz, δ (ppm), D₂O]: 4.25-4.15 (br, 2 H, 7-C*H*₂), 3.80-3.45 (br, 10 H, 1-C*H*₂ + 6-C*H*₂), 2.75-2.60 (br, 6 H, 5-C*H*₂ + 8-C*H*₂), 2.60-2.45 (br, 2 H, 4-C*H*₂), 2.40-2.25 (br, 2 H, 2-C*H*₂), 1.15-1.00 (br, 3 H, 3-C*H*₃). Experimentally determined copolymer ratio: m/o 70:30. **MALDI-TOF:** Mₙ 13.5 kDa. **Yield:** 81%.

### P(EtOx₇₀-NHS₃₀) P1d

According to the general procedure, the reaction of a solution of **P1c** (61 g, 3.91 mmol), *N-*hydroxysuccinimide (14.85 g, 129.00 mmol) and *N*,*N'*-diisopropylcarbodiimide (21.79 mL, 140.72 mmol) in DCM/ACN 9:1 (1346 mL, 0.2 M) afforded the desired polymer P1d (57.06 g, mmol). **¹H NMR** [400 MHz, δ (ppm), DMSO-d₆]: 4.00-3.90 (br, 2 H, 7-C*H*₂), 3.50-3.10 (br, 10 H, 1-C*H*₂ + 6-C*H*₂), 2.90-2.85 (br, 2 H, 8-C*H*₂), 2.75-2.70 (br, 4 H, 9-C*H*₂), 2.65-2.60 (br, 2 H, 8-C*H*₂), 2.60-2.40 (br, 2 H, 5-C*H*₂), 2.30-2.10 (br, 4 H, 2-C*H*₂ + 4-C*H*₂), 0.85-0.70 (br, 3 H, 3-C*H*₃). Experimentally determined copolymer ratio: m/o 70:30. **SEC:** Mₙ 15.6 kDa, Ð 1.25. **MALDI-TOF:** Mₙ 16.9 kDa. **Yield:** 79%.

### P(EtOx₇₀-Ale₃₀) P1e

According to the general procedure, the reaction of a solution of P1d (15 g, 0.81 mmol), sodium alendronate trihydrate (15.80 g, 48.61 mmol), *N*-hydroxysuccinimide (2.80 g, 24.31 mmol) and *N*-(3-dimethylaminopropyl)-*N'*-ethylcarbodiimide hydrochloride (4.66 g, 24.31 mmol) in PBS (66 mL, 0.5 M) afforded the desired polymer **P1e** (5.81 g, 0.26 mmol). **¹H NMR** [400 MHz, δ (ppm), D₂O]: 4.25-4.10 (br, 4 H, 7-C*H*₂), 3.80-3.30 (br, 16 H, 1-C*H*₂ + 6-C*H*₂), 3.25-3.15 (br, 2 H, 9-C*H*₂), 2.75-2.45 (br, 16 H, 4-C*H*₂ + 5-C*H*₂ + 8-C*H*₂), 2.45-2.15 (br, 2 H, 2-C*H*₂), 2.15-1.70 (br, 4 H, 10-C*H*₂ + 11-C*H*₂), 1.15-0.95 (br, 3 H, 3-C*H*₃). **³¹P NMR** [400 MHz, δ (ppm), D₂O]: 18.25. Experimentally determined copolymer ratio: m/o/p 70:2:28. **MALDI-TOF:** Mₙ 19.5 kDa. **Yield:** 32%.

### Example 2

### Synthesis of P(EtOx₇₀-OH₁₀-Ale₂₀) - P2e

### P(EtOx₇₀-OH₁₀-COOH₂₀) P2c

According to the general procedure, the reaction of a solution of **P1b** (15 g, 1.19 mmol), 4-dimethylamino pyridine (0.58 g, 4.76 mmol) and succinic anhydride (2.86 g, 28.54 mmol) in DCM/ACN 9:1 (17 mL, 2 M) afforded the desired polymer **P2c** (17.33 g, 1.19 mmol). **¹H NMR** [400 MHz, δ (ppm), D₂O]: 4.25-4.15 (br, 2 H, 9-C*H*₂), 3.80-3.45 (br, 16 H, 1-C*H*₂ + 7-C*H*₂ + 8-C*H*₂), 3.35-3.25 (br, 2 H, 6-C*H*₂), 2.75-2.60 (br, 8 H, 5-C*H*₂ + 10-C*H*₂), 2.60-2.45 (br, 4 H, 4-C*H*₂), 2.40-2.25 (br, 2 H, 2-C*H*₂), 1.15-1.00 (br, 3 H, 3-C*H*₃). Experimentally determined copolymer ratio: m/n/o 70:12:18. **MALDI-TOF:** Mₙ 12.5 kDa. **Yield:** 94%.

### P(EtOx₇₀-OH₁₀-NHS₂₀) P2d

According to the general procedure, the reaction of a solution of **P2c** (12 g, 0.82 mmol), *N-*hydroxysuccinimide (2.08 g, 18.07 mmol) and *N*,*N*'-diisopropylcarbodiimide (3.05 mL, 19.72 mmol) in DCM/ACN 9:1 (190 mL, 0.2 M) afforded the desired polymer **P2d** (11.95 g, 0.72 mmol). **¹H NMR** [400 MHz, δ (ppm), DMSO-d₆]: 4.15-4.00 (br, 2 H, 8-C*H*₂), 3.75-3.40 (br, 16 H, 1-C*H*₂ + 6-C*H*₂ + 7-C*H*₂), 3.35-3.25 (br, 2 H, 5-C*H*₂), 3.05-2.95 (br, 2 H, 9-C*H*₂), 2.95-2.85 (br, 4 H, 10-C*H*₂), 2.85-2.70 (br, 2 H, 9-C*H*₂), 2.75-2.65 (br, 8 H, 4-C*H*₂), 2.40-2.10 (br, 2 H, 2-C*H*₂), 1.05-0.95 (br, 3 H, 3-C*H*₃). Experimentally determined copolymer ratio: m/n/o 70:12:18. **SEC:** Mₙ 14.7 kDa, Ð 1.25. **MALDI-TOF:** Mₙ 14.4 kDa. **Yield:** 88%.

### P(EtOx₇₀-OH₁₀-Ale₂₀) P2e

According to the general procedure, the reaction of a solution of **P2d** (12 g, 0.74 mmol), sodium alendronate trihydrate (8.69 g, 6.74 mmol), *N*-hydroxysuccinimide (1.54 g, 13.37 mmol) and *N*-(3-dimethylaminopropyl)-*N'*-ethylcarbodiimide hydrochloride (2.56 g, 13.37 mmol) in PBS (108 mL, 0.5 M) afforded the desired polymer **P2e** (4.15 g, 0.23 mmol). **¹H NMR** [400 MHz, δ

(ppm), D₂O]: 4.30-4.10 (br, 4 H, 8-C*H*₂), 3.80-3.40 (br, 22 H, 1-C*H*₂ + 6-C*H*₂ + 7-C*H*₂), 3.40-3.30 (br, 2 H, 5-C*H*₂), 3.30-3.20 (br, 2 H, 10-C*H*₂), 2.75-2.15 (br, 22 H, 2-C*H*₂ + 4-C*H*₂ + 9-C*H*₂), 2.05-1.75 (br, 4 H, 11-C*H*₂ + 12-C*H*₂), 1.15-0.95 (br, 3 H, 3-C*H*₃). **³¹P NMR** [400 MHz, δ (ppm), D₂O]: 18.25. Experimentally determined copolymer ratio: m/n/o/p 70:12:2:16. **MALDI-TOF:** Mₙ 14.6 kDa. **Yield:** 30%.

### Example 3

### Preparation of water-soluble electrophilic polymer (P(EtOx₆₀-OH₂₀-NHS₂₀))

### Preparation of P(EtOx₆₀-OH₂₀-NHS₂₀)

NHS-side chain activated poly[2-(ethyl/hydroxy-ethyl-amide-ethyl/NHS-ester-ethyl-ester-ethyl-amide-ethyl)-2-oxazoline] terpolymer containing 20% NHS-ester groups (= EL-POx, 20% NHS) was synthesized as follows:
Poly[2-(ethyl/methoxy-carbonyl-ethyl)-2-oxazoline] copolymer (DP = +/-100) was synthesized by means of CROP using 60% 2-ethyl-2-oxazoline (EtOx) and 40% 2-methoxycarbonyl-ethyl-2-oxazoline (MestOx). A statistical copolymer containing 40% 2-methoxycarbonyl-ethyl groups (¹H-NMR) was obtained. Secondly, the polymer containing 40% 2-methoxycarbonyl-ethyl groups, was reacted with ethanolamine yielding a copolymer with 40% 2-hydroxy-ethyl-amide-ethyl-groups (¹H-NMR). After that, half of the 2-hydroxy-ethyl-amide-ethyl-groups was reacted with succinic anhydride yielding a terpolymer with 60% 2-ethyl groups, 20% 2-hydroxy-ethyl-amide-ethyl-groups and 20% 2-carboxy-ethyl-ester-ethyl-amide-ethyl-groups according to ¹H-NMR. Lastly, the 2-carboxy-ethyl-ester-ethyl-amide-ethyl-groups were activated by N-hydroxysuccinimide (NHS) and diisopropylcarbodiimide (DIC), yielding EL-POx, 20% NHS. The NHS-POx contained 20% NHS-ester groups according to ¹H-NMR. NHS-POx was dissolved between 2-8 °C in water (60 g in 300 mL), cooled at minus 80 °C for half an hour and freeze dried. The freeze dried powder so obtained was dried in a Rotavap at 40 °C until the water content was below 0.8% w/w as determined via Karl Fischer titration. This dry (white) powder was grinded using a ball mill (Retch MM400) until the average particle size was not more than 40 µm (D [4,3]) and vacuum sealed in alu-alu bags.

### Example 4

### Characterization of Alendronate-functionalized Poly(2-oxazoline)s

The degree of modification of the different substitutions in the polymers was determined by ¹H NMR. Moreover, the presence of alendronate moieties by ³¹P NMR. NMR spectra was recorded on a Varian Inova 400 (400 MHz) or Bruker Avance III (400 MHz) spectrometer in the indicated solvent at 25 °C. ¹H NMR data is reported as: chemical shifts (given in parts per million (ppm) with respect to tetramethylsilane as standard), multiplicity (br = broad), integration, and assignment. The number average molecular weights (Mₙ) was recorded on a Bruker Microflex LRF Matrix-assisted laser desorption ionisation time-of-flight mass spectrometry (MALDI-TOF MS) system. All mass spectra were obtained in positive ion mode. α-Cyano-4-hydroxycinnamic acid (CHCA) was used as a matrix in tetrahydrofuran (10 mg/mL). Polymer samples were dissolved in THF:methanol v/v 1:1 (10 mg/mL) and analyte solutions were prepared by mixing 10 µL of matrix and 1 µL of polymer sample. Samples were applied using the dried droplet method. Size exclusion chromatography (SEC) was performed on an automated Shimadzu HPLC system, with a PLgel 5 µm MIXED-D column at 50 °C, using *N*,*N-*dimethylacetamide (DMA) containing 50 mM LiCI as eluent at a flow rate of 0.6 mL/min. Polydispersity index values (PDI) were calculated against poly(methyl methacrylate) (PMMA) standards.

The results of these analyses are summarized in Table 1.

**Table 1**

| | **¹H NMR (mol %)** | | | | | **Mₙ (kDa)** | | |
|---|---|---|---|---|---|---|---|---|
| | **EtOx** | **OH** | **COOH** | **Ale** | **Conversion** (%) | **Theor.^{a}** | **MALDI-TOF** | **PDI** |
| **P1e** | 70 | | 2 | 28 | 93 | 19.1 | 19.5 | 1.11 |
| **P2e** | 70 | 12 | 2 | 16 | 89 | 15.5 | 14.6 | 1.11 |

| | | | | | | | | |
|---|---|---|---|---|---|---|---|---|
| *Theoretical values were calculated considering the polymerization degree of MestOx-functionalized polymers obtained by MALDI-TOF and functionality ratios by ¹H NMR.* | | | | | | | | |

### Example 5

### Isothermal Titration Calorimetry (ITC)

Isothermal titration calorimetry was used to assess the affinity between the synthesized POx-Ale polymers and dissolved calcium cations in water. ITC experiments were carried out on a fully automated Microcal Auto-iTC200. Curve fitting was performed by Origin 6.0 using one set of sites binding model. Generally, 0.36 mM per unit of alendronate present in the polymer were titrated with 4 mM CaCl₂ in Mili-Q water at 20 °C. All polymers were titrated with the same batch of calcium. Each ITC titration consisted of 19 injections. All measurements were performed in triplicate.

The statistical analyses were performed using GraphPad InStat^{®} software. Rheological results were analysed statistically using a one-way ANOVA test, followed by Tukey's multiple comparison test. The significance threshold was set at P < 0.05.

The results of the ICT analyses are summarized in Table 2.

**Table 2**

| | **Stoichiometry, *N*** | **K_{ca}²⁺ (M⁻¹)** | **ΔH (cal mol⁻¹)** | **ΔS (cal deg⁻¹ mol⁻¹)** |
|---|---|---|---|---|
| **Alendronate** | 0.5 ± 0.0 | 7.8 × 10³ ± 1.8 × 10³ | 1.3 × 10³ ± 8.5 × 10² | 22.4 ± 2.3 |
| **P1e** | 25.6 ± 2.4 | 2.4 × 10⁵ ± 4.0 × 10³ | 1.5 × 10³ ± 2.4 × 10² | 29.6 ± 0.8 |
| **P2e** | 15.1 ± 0.2 | 1.2 × 10⁵ ± 3.6 × 10³ | 1.1 × 10³ ± 2.7 × 10² | 27.1 ± 0.9 |

### Example 6

### Preparation of calcium-crosslinked POx-Ale gels

Gels were prepared mixing equal volumes of POx-Ale in phosphate buffer saline (PBS) at different polymer concentration (10, 20, 30 wt.%) with several CaCl₂ solutions in miliQ water (1, 10, 20, 40 wt.%), with a total volume of 200 µL. The mixtures were stirred vigorously using vortex for 15 sec in order to get homogenous gels.

### Visual Observation of Gelation

Mixing equal volumes of alendronate-functionalized polymers and Ca²⁺ solutions at final polymer concentrations of 10, 20 and 30 wt.%, and Ca²⁺ concentrations between 1 and 40 wt.% yielded either viscous solutions or self-healing hydrogels. Generally, increasing both polymer and calcium concentrations formed stronger gels.

Macroscopically, two different kind of gels were created: P(EtOx₇₀-Ale₃₀) produced a white stable transient network, whereas P(EtOx₇₀-OH₁₀-Ale₂₀) formed transparent soft gels. When more than 20 mol % alendronate was present in the polymer, the binding affinity was so strong that the gelation process competed with polymer precipitation, such precipitation causing the gel to lose its transparency.

The self-healing properties of a hydrogel (30 wt.% P(EtOx₇₀-OH₁₀-Ale₂₀) and 20 wt.% CaCl₂) was evaluated visually. Two gels of identical composition were coloured using either blue (Brilliant) or red (Amaranth) dyes. They were cut transversally in half, and then two differently dyed halves colour were brought together without applying external force. After two minutes of contact, no border between them was observed, and their connexion was strong enough to allow for stretching.

### In Vitro Stability of the Hydrogels

In order to study both the stability and the reversibility of the formed crosslinks, the gels were soaked in EDTA (100 mM, pH 6) and they were monitored for 48 h.

P(EtOx₇₀-OH₁₀-Ale₂₀) was completely dissolved in EDTA within 3 h of immersion, which confirmed that the crosslinks between Ca²⁺ and alendronate were responsible for the network formation. However, P(EtOx₇₀-Ale₃₀) remained stable even after 48 h of soaking time in EDTA, indicating that the crosslinks were strong enough not to be disrupted by the binding agent.

### Example 7

### Preparation of bone- and mineral-adhesive sheets

Adhesive sheets were prepared by dry deposition of adhesive polymers (P(EtOx₇₀-Ale₃₀) and P(EtOx₇₀-OH₁₀-Ale₂₀) into commercially available fibrous gelatin carrier (GELITA TUFT-IT^{®}, Germany) of 5 × 7 cm², followed by application of a backing layer. Alendronate-free control sheets were prepared by dry deposition of P(EtOx) into the same fibrous gelatin carrier, again followed by application of a backing layer.

The fibrous gelatin carrier, which consisted of 8 cohesive layers, was delaminated to obtain carriers of 3 layers of approximately 300 mg and 0.3 mm thick.

The polymers were processed into fine powders by ball milling at 30 Hz for 10 min, followed by sieving below 63 µm. Next, the polymers were dried using a rotary evaporator at 40 °C, 2kPa (20 mbar) for 16 h and the carrier was dried using a vacuum oven at 40 °C, 0.5 kPa (5 mbar) for 16 h.

The dry polymer powders were placed into a grid array of 5 × 7 × 0.4 cm³, consisting of 726 square wells, and the carriers were placed on top of the array, surrounded by a spacing frame of 1.5 mm thickness. The weight ratio polymer:carrier was 65:35. Next, the array was positioned between two dielectric plates and polymers were loaded into the carriers using a high voltage electrostatic impregnation system (Fibroline SL-Preg, France) at 40 kV, 100 Hz for 20 sec, with a loading efficacy of approximately 80%, obtaining a homogenous distribution of the polymer powders through the carriers.

After impregnation, a backing layer was placed on top of the impregnated carriers and anti-adhesive papers were placed underneath and on top of the combination before it was fixated to a heating plate with the backing layer facing the heating plate. Adhesion of the backing layer to the impregnated carrier was achieved by two cycles of heating while compressing at 150 °C, 30 N for 3 sec, obtaining a final weight ratio of polymer/carrier/backing 42%:28%:30%. The sheets so obtained were cut using a scalpel (carbon steel, blade 20) into the required dimensions for each experiment. Further, they were placed individually in aluminum bags and dried at 40 °C, 0.5 kPa (5 mbar) for 16h. Finally, the bags were sealed at 0.2 kPa (2 mbar) for 4 sec with a nitrogen flush of 60 kPa (600 mbar).

The physical properties of the sheets (1.5 × 2.5 cm²), i.e. thickness and density, are summarized in Table 3.

**Table 3**

| | Thickness (mm) | Density (g/cm³) |
|---|---|---|
| Carrier | 0.28 ± 0.02 | 0.31 ± 0.08 |
| Backing | 0.05 ± 0.00 | 1.70 ± 0.08 |
| Carrier + backing | 0.28 ± 0.02 | 0.39 ± 0.03 |
| P(EtOx) | 0.31 ± 0.02 | 0.58 ± 0.04 |
| P(EtOx₇₀-Ale₃₀) | 0.25 ± 0.03 | 0.55 ± 0.07 |
| P(EtOx₇₀-OH₁₀-Ale₂₀) | 0.31 ± 0.04 | 0.50 ± 0.04 |

### Preparation of bone samples

Porcine ribs were obtained and sectioned using a circular saw machine into rectangular pieces of approximately 2.5 × 0.7 × 1.2 cm³. Each piece of bone was polished (Struers TegraPol 35, USA) using carbon paper (P500) at 150 rpm until a flat surface of cortical bone was exposed. Thereafter, the samples were kept frozen at -20 °C until further use.

Flat bone specimens were immersed in 500 mL of Sakura reagent TDE^{™} 30 and decalcified using the Sakura TDE^{™} 30 electrode system for 24 h. Thereafter, they were kept frozen at -20 °C until further use.

### Preparation of apatite-coated titanium plates

Commercially pure titanium plates (2.5 × 0.1 × 2.5 cm³, grade 2) were first grit-blasted to obtain an average roughness Rₐ of 1.5µm. Subsequently, these substrates were ultrasonically cleaned with Milli-Q water, followed by isopropanol during 20 min. Next, the substrates were placed on a rotating holder and etched with argon plasma for 10 min prior to physical vapor deposition of a thin adherent coating of hydroxyapatite using a radiofrequent magnetron sputtering system (Edwards ESM 100), at a power of 400 W and an argon pressure of 0.0005 kPa (5 × 10⁻³ mbar) for 12 h. After deposition, the hydroxyapatite-coated plates were crystallized by a heating treatment in a furnace at 650 °C for 2 h, with a heating and cooling rate of 1.5 °C/min, yielding an apatitic calcium phosphate coating of 1 µm.

### Tensile tests

Tensile tests were performed using a tensile bench (LS5, Lloyd Instruments, UK) equipped with a 100 N load cell. All measurements were performed in sextuplicate (n = 6). The aforementioned sheets were cut into pieces of 1.5 × 2.5 cm² (length × height).

Each end of the sheets was fixed to the tensile grips, and the sheets were subsequently tested in dry state at a crosshead displacement speed of 1 mm/min until breakage. The tensile force was recorded as a function of displacement, and the tensile force vs. sheet extension was acquired automatically. The tensile strength was calculated as the maximum load before breakage of the samples divided by their cross-sectional area. The tensile modulus of the sheets was calculated as the average slope of initial linear part of the stress-strain curves.

The tensile strength and the modulus of the different sheets are depicted in Table 4.

**Table 4**

| **Sheet** | **Tensile strength (in MPa)** | **Tensile modulus (in MPa)** |
|---|---|---|
| Carrier (GELITA TUFT-IT^{®}) | 0.74 ± 0.20 | 13.32 ± 3.36 |
| Carrier + backing | 2.03 ± 0.31 | 41.95 ± 8.89 |
| Carrier + backing impregnated with P(EtOx₇₀-Ale₃₀) | 2.25 ± 0.21 | 44.29 ± 6.94 |
| Carrier + backing impregnated with P(EtOx₇₀-OH₁₀-Ale₂₀) | 3.86 ± 0.72 | 76.22 ± 14.65 |

The application of hydroxyl-functionalized POx polymer onto the sheets composed of fibrous gelatin and polyester backing further reinforced these sheets. This phenomenon may be attributable to interactions between hydroxyl groups of the polyoxazoline and amines and carboxylic acid groups present in the carrier, but also with the amines present in the backing layer.

### Lap-shear adhesion tests to apatite-coated substrates, apatite-free control substrates, bone and demineralized bone specimens

Lap-shear adhesion tests were performed using a tensile bench (LS5, Lloyd Instruments, UK) equipped with a 100 N load cell. All measurements were performed in sextuplicate (n = 6). To measure the adhesion to apatite-coated substrates, the sheets were cut into pieces of 1.5 × 2.5 cm². The sheets were cut into pieces of 1.5 × 1.0 cm² for the adhesion tests onto bone, since the bone samples were smaller. Uncoated titanium and demineralized bone specimens were used as control model surfaces.

Sheets were glued to plastic holders. Next, substrates were moistened with PBS soaked gauze followed by controlled attachment of the sheets onto the substrates at a force of 10 N for 5 min. Next, lap-shear adhesion tests were carried out at a crosshead displacement speed of 0.5 mm/min. The shear strength was calculated by dividing the maximum load before breakage by their overlapping contact surface of 3.75 cm² for Ti plates and 1.5 cm² for bone specimens.

The tensile modulus of the sheets was calculated as the average slope of initial linear part of the stress-strain curves.

The results of the lap-shear adhesion measurements are summarized in Table 5.

**Table 5**

| | **Shear strength (in kPa)** | | | |
|---|---|---|---|---|
| **Sheet** | **Titanium** | **CaP coated titanium** | **Bone** | **Demineralized bone** |
| Carrier + backing | 4.44 ± 6.37 | 3.99 ± 2.87 | 2.92 ± 1.61 | 0.66 ± 0.32 |
| Carrier + P(EtOx₇₀-Ale₃₀) + backing | 4.64 ± 5.27 | 42.24 ± 11.36 | 25.85 ± 12.56 | 0.61 ± 0.15 |
| Carrier + P(EtOx₇₀-OH₁₀-Ale₂₀) + backing | 2.14 ± 3.32 | 35.88 ± 12.60 | 19.66 ± 12.27 | 1.11 ± 0.70 |
| Bio-Gide^{®} | 0.94 ± 0.91 | 1.01 ± 1.22 | 1.34 ± 0.67 | 2.47 ± 1.20 |

The polymer with the highest number of alendronate moieties (P(EtOx₇₀-Ale₃₀)) exhibited an adhesion to CaP coated-Ti plates that was 41 times higher, and an adhesion to bone that was 18 times higher than that of Bio-Gide^{®}, a barrier membrane widely used for dental applications.

### Underwater adhesion tests to bone

Sheets of 1 cm² were adhered to bone specimens for 5 minutes. Next, they were immersed in 200 mL of water, stirred at 150 rpm and monitored for 24 hours to evaluate their adhesion and swelling. Experiments were done in triplicate.

A summary of the results is shown in Table 6. Degree of swelling was scored as:
3 = high swelling
2 = medium swelling
1 = low swelling

Degree of adhesion was scored as:
- = no adhesion by any of the sheets
+/- = poor adhesion
+ = good adhesion
++ = excellent adhesion by all 3 sheets

**Table 6**

| **Sheet** | **Swelling** | **Adhesion to bone** |
|---|---|---|
| Carrier + backing | 3 | - |
| Carrier + P(EtOx₇₀-Ale₃₀) + backing | 1 | ++ |
| Carrier + P(EtOx₇₀-OH₁₀-Ale₂₀) + backing | 2 | + |
| Carrier + P(EtOx) + backing | 3 | - |

Only the sheets containing POx-alendronate remained adhesive to bone after immersion in water for 24 hours. Moreover, these sheets showed less swelling than the other sheets.

### Example 8

### Ex-vivo model

The Lung Assist (Organ Assist, Groningen, the Netherlands) perfusion device was used as the extra corporal organ perfusion system. This device, which is depicted in Figure 3, consists of two blood reservoirs (1) and (2), an automatic centrifugal pump system (3) to provide arterial flow and pressure, a (de-)oxygenator (4), a heater/cooler unit with an inlet (5) and outlet (6) for cooling/heating medium, a flow sensor (7), temperature sensor (8) and warm water bath holding a porcine cadaveric head (9).

Heparinized full blood was used to prime the system. The system was running for several minutes to heat up and to stabilize the hemodynamic parameters. The porcine cadaveric head was placed in a box filled with warm water (37 °) that was connected to a laboratory water bath. Subsequently, cannulation of both the common carotid arteries was performed. Cannulas were filled with blood to remove as much air as possible from the system, and the extracorporeal system was connected to the porcine head. One side of the porcine head was heated from the water from the water bath for approximately 30 minutes, thereafter the porcine head was turned to initiate the surgical procedure on the heated side. After performing the surgery, the head was turned again and the procedure was repeated on the other side of the mandibula. Heparinized blood was oxygenated using 95% oxygen and 5% carbon dioxide. Point-of-care (PoCT) hematology tests (pH, PCO₂, PO₂, BE_{ecf}, HCO₃, TCO₂, sO₂, Hb Hematocrit, Na, K, iCa, Glu and Activated Clotting Time (ACT)) were performed using the i-STAT (Abbott Laboratories, Abbott Park, IL, USA) analyzer. Hemodynamic parameters (PoCT tests, pressures and flows) were measured every ~1.5h.

### Preparation of bone- and mineral-adhesive sheets

Adhesive sheets were prepared by dry deposition of adhesive polymers (P(EtOx₇₀-Ale₃₀), P(EtOx₇₀-OH₁₀-Ale₂₀) and P(EtOx₆₀-OH₂₀-NHS₂₀) into commercially available fibrous gelatin carrier (GELITA TUFT-IT^{®}, Germany) of 5 × 7 cm², followed by application of a backing layer. The fibrous gelatin carrier, which consisted of 8 cohesive layers, was delaminated to obtain carriers of 3 layers of approximately 300 mg and 0.3 mm thick.

The polymers were processed into fine powders by ball milling at 30 Hz for 10 min, followed by sieving below 63 µm. Next, the polymers were dried using a rotary evaporator at 40 °C, 2 kPa (20 mbar) for 16 h and the carrier was dried using a vacuum oven at 40 °C, 0.5 kPa (5 mbar) for 16 h.

In order to prepare impregnated carriers containing a mixture of either P(EtOx₇₀-Ale₃₀) and P(EtOx₆₀-OH₂₀-NHS₂₀) 1:1 or P(EtOx₇₀-OH₁₀-Ale₂₀) and P(EtOx₆₀-OH₂₀-NHS₂₀) 1:1, these polymer mixtures were granulated by adding 3 wt % of acetone, followed by drying in a rotatory evaporator at 40 °C, 2 kPa (20 mbar) for 16 h.

The dry polymer powders were placed into a grid array of 5 × 7 × 0.4 cm², consisting of 726 square wells, and the carriers were placed on top of the array surrounded with a spacing frame of the 1.5 mm thickness, in a weight ratio of polymer/carrier 65:35. Next, the array was positioned between two dielectric plates and polymers were loaded into the carriers using a high voltage electrostatic impregnation system (Fibroline SL-Preg, France) at 40 kV, 100 Hz for 20 sec, with a loading efficacy of approximately 80%, obtaining a homogenous distribution of the polymer powders through the carriers.

After impregnation, a backing layer was placed on top of the impregnated carriers and anti-adhesive papers were placed underneath and on top of the combination before it was introduced into a heating plate. Adhesion of the backing layer to the impregnated carrier was achieved by two cycles of heating while compressing at 150 °C, 30 N for 3 sec, obtaining a final weight ratio of polymer/carrier/backing 42%:28%:30%.

The sheets so obtained were cut using a scalpel (carbon steel, blade 20) into the required dimensions for each experiment. Further, they were placed individually in aluminum bags and dried at 40 °C, 0.5 kPa (5 mbar) for 16 h. Finally, the bags were sealed at 0.2 kPa (2 mbar) for 4 sec with a nitrogen flush of 60 kPa (600 mbar).

### Ex-vivo adhesion tests

Five cadaveric porcine heads were prepared for experiments. A submandibular incision was made, parallel to the inferior border of mandible. Subsequently, the periosteum was carefully elevated. In total, a maximum of five standardized bone defects (ø3.35mm) were created using a dental drill, cooled with saline solution. All defects were filled with bone debris and, if necessary, supplemented with Bio-Oss (Geistlich Pharma AG, Wolhunsen, Switzerland) mixed with heparinized blood, and subsequently covered with a bone adhesive sheet (10x10 mm). The sheet was manually pressed for 30 seconds, and left for 4.5 minutes until the adhesive tests were performed.

Six sheets (see Example 7) were tested in sextuplicate (n = 6) and all 36 sheets were randomized before testing.

Degree of adhesion was scored as: no adhesion, poor adhesion, moderate adhesion, and strong adhesion. A summary of the results is shown in Table 7. In the table, numbers represent the number of membranes tested.

**Table 7**

| **Sheet** | **No adhesion** | **Poor adhesion** | **Moderate adhesion** | **Strong adhesion** |
|---|---|---|---|---|
| Carrier + backing | 2 | 3 | 1 | |
| Carrier + P(EtOx₇₀-Ale₃₀) + backing | 1 | 1 | 3 | 1 |
| Carrier + P(EtOx₇₀-OH₁₀-Ale₂₀) + backing | 1 | 1 | 3 | |
| Carrier + P(EtOx₆₀-OH₂₀-NHS₂₀) + backing | | | 1 | 5 |
| Carrier + P(EtOx₇₀-Ale₃₀) + P(EtOx₆₀-OH₂₀-NHS₂₀) + backing | | | 1 | 5 |
| Carrier + P(EtOx₇₀-OH₁₀-Ale₂₀) + P(EtOx₆₀-OH₂₀-NHS₂₀) + backing | | 1 | 3 | 2 |

### Example 9

### Synthesis of PEG-Ale₈ Mw ~ 10,000

Using the same procedure as used for the synthesis of alendronate-functionalized poly(2-oxazoline)s, alendronate-functionalized polyethylene glycol (2.97 g, 0.26 mmol) was prepared using 8-arm PEG-NHS (5.0 g, 0.5 mmol), sodium alendronate trihydrate (1.63 g, 6 mmol), N-hydroxysuccinimide (460 mg, 4 mmol) and *N*-(3-dimethylaminopropyl)-*N'*-ethylcarbodiimide hydrochloride (767 mg, 4 mmol) in PBS (25 mL, 0.5 M). **¹H NMR** [400 MHz, δ (ppm), D₂O]: 4.10 (s, 16 H), 3.96 (d, 6H) 3.87-3.58 (br, 1168 H), 2.09-1.70 (br, 32 H). **³¹P NMR** [400 MHz, δ (ppm), D₂O]: 18.04. **Yield:** 54%

### Example 10

### Preparation of granulate comprising P(EtOx₆₀-OH₂₀-NHS₂₀) and alendronic acid

P(EtOx₆₀-OH₂₀-NHS₂₀) (4.00 g, 0.29 mmol) and alendronic acid (1.44 g, 5.78 mmol) were placed in a high-shear mill. While milling, acetone (0.8 mL) was added dropwise. Subsequently, the granulate was dried followed by drying in a rotatory evaporator at 40 °C, 2 kPa (20 mbar) for 16 h.

### Preparation of granulate comprising PEG-NHSs and alendronic acid

PEG-NHSs (4.00 g, 0.40 mmol) and alendronic acid (0.79 g, 3.17 mmol) were placed in a high-shear mill. While milling, acetone (0.4 mL) was added dropwise. Subsequently, the granulate was dried followed by drying in a rotatory evaporator at 25 °C, 2 kPa (20 mbar) for 16 h.

### Example 11

### Preparation of bone- and mineral-adhesive sheets

Adhesive sheets without a backing layer were prepared by dry deposition of adhesive polymers and adhesive granulates into commercially available fibrous gelatin carrier (GELITA TUFT-IT^{®}, Germany) of 5 × 7cm² (see Example 7).

The average particle size (D[4,3]) of the adhesive polymers and adhesivie granulates used in the preparation of the adhesive sheets are shown in Table 8.

**Table 8**

| | **D[4,3] (µm)** |
|---|---|
| P(EtOx₆₀-OH₂₀-Ale₂₀) | 112.5 |
| P(EtOx₆₀-OH₂₀-NHS₂₀) / alendronic acid granulate | 39.5 |
| PEG-Ale₈ | 176.0 |
| PEG-NHS₈ / alendronic acid granulate | 299.6 |

### Lap-shear adhesion tests to apatite-coated titanium

Lap-shear adhesion tests to apatite-coated titanium were performed as in Example 7. The results of the lap-shear adhesion measurements are summarized in Table 9.

**Table 9**

| **Sheet** | **Shear strength (in kPa) CaP coated titanium** |
|---|---|
| Carrier + P(EtOx₆₀-OH₂₀-Ale₂₀) | 47.5 ± 26.7 |
| Carrier + P(EtOx₆₀-OH₂₀-NHS₂₀) / alendronic acid qranulate | 13.9 ± 0.86 |
| Carrier + PEG-Ale₈ | 17.9 ± 1.43 |
| Carrier + PEG-NHS₈ / alendronic acid granulate | 35.5 ± 2.57 |

The adhesion results for the sheet containing a granulate of P(EtOx₆₀-OH₂₀-NHS₂₀) and alendronic acid can be improved considerably by including a carbonate/bicarbonate buffer (1:1 mole/mole, pH 8,5) in the granulate and/or by employing a coarser granulate.

## Claims

1. A biocompatible, flexible, bone-adhesive sheet comprising:
• a cohesive fibrous carrier structure comprising a three-dimensional interconnected interstitial space; and
• distributed within the interstitial space, (i) a plurality of polymer particles comprising a water-soluble calcium-binding polymer selected from polyoxazolines, polyethylene glycols, polyvinylpyrrolidones, polyurethanes and combinations thereof, said water-soluble calcium-binding polymer carrying at least one calcium-binding group selected from bisphosphonate, citrate, ethylenediaminetetraacetic acid (EDTA) and combinations thereof or (ii) a plurality of reactive particles comprising an electrophilically activated water-soluble polymer selected from polyoxazolines, polyethylene glycols, polyvinylpyrrolidones, polyurethanes and combinations thereof and a plurality of bisphosphonate particles comprising nitrogenous bisphosphonate,
wherein calcium-binding group is a group that is capable of forming two or more separate coordinate bonds with Ca²⁺ ions.

2. Bone-adhesive sheet according to claim 1, wherein the fibres in the fibrous carrier structure have a mean diameter of 1-500 µm.

3. Bone-adhesive sheet according to any one of the preceding claims, wherein the fibrous carrier structure has a felt structure, a woven structure or a knitted structure, preferably a felt structure.

4. Bone-adhesive sheet according to any one of the preceding claims, wherein the fibrous carrier structure comprises at least 50 wt.% fibers containing gelatin, collagen, cellulose, modified cellulose, carboxymethyldextran, PLGA, sodium hyaluronate/carboxy methylcellulose, polyvinyl alcohol, chitosan or a combination thereof.

5. Bone-adhesive sheet according to any one of the preceding claims, wherein the bone-adhesive sheet comprises, distributed within the interstitial space, a plurality of polymer particles comprising a water-soluble calcium-binding polymer, said water-soluble calcium-binding polymer carrying at least one calcium-binding group.

6. Bone-adhesive sheet according to claim 5, wherein the fibrous carrier structure contains 5-90% polymer particles, said percentage being calculated by weight of the fibrous carrier structure.

7. Bone-adhesive sheet according to claim 5 or 6, wherein the polymer particles contain at least 10 wt.% of the water-soluble calcium-binding polymer.

8. Bone-adhesive sheet according to any one of claims 5-7. wherein the polymer particles have a volume weighted mean diameter in the range of 10-100 µm.

9. Bone-adhesive sheet according to any one of claims 5-8, wherein the calcium-binding groups comprise bisphosphonate.

10. Bone-adhesive sheet according to any one of claims 1-4, wherein the bone-adhesive sheet comprises, distributed within the interstitial space, a plurality of the reactive particles comprising an electrophilically activated water-soluble polymer and a plurality of the bisphosphonate particles comprising nitrogenous bisphosphonate.

11. Bone-adhesive sheet according to claim 10, wherein the fibrous carrier structure contains 5-90% of the combination of the reactive particles and the bisphosphonate particles, said percentage being calculated by weight of the fibrous carrier structure.

12. Bone-adhesive sheet according to claim 10 or 11, wherein the reactive particles and the bisphosphonate particles are combined in agglomerates.

13. Bone-adhesive sheet according to claim 12. wherein the agglomerates of reactive particles and bisphosphonate particles have a volume weighted mean diameter D [4,3] in the range of 100-400 µm.

14. Bone-adhesive sheet according to any one of claims 10-13, wherein the reactive particles contain at least 10 wt.% of the electrophilically activated water-soluble polymer.

15. A method of preparing a bone-adhesive sheet according to any one of the preceding claims, said method comprising:
• providing a water-resistant cohesive fibrous carrier structure comprising a three-dimensional interconnected interstitial space;
• providing polymer particles comprising a water-soluble calcium-binding polymer, said water-soluble calcium-binding polymer carrying at least one calcium-binding group or providing reactive particles comprising an electrophilically activated water-soluble polymer and bisphosphonate particles comprising nitrogenous bisphosphonate;
• impregnating the fibrous carrier structure with the polymer particles or impregnating the fibrous carrier structure with both the reactive particles and the bisphosphonate particles.

16. Method according to claim 15, wherein the fibrous carrier structure is impregnated by:
• placing the fibrous carrier structure and the polymer particles between two electrodes;
• simultaneously subjecting the fibrous carrier structure and the polymer particles to an electric field of 0.1 to 40 kV/mm to impregnate the interconnected interstitial space of the fibrous carrier structure with the polymer particles or simultaneously subjecting the fibrous carrier structure, the reactive particles and the bisphosphonate particles to an electric field of 0.1 to 40 kV/mm to impregnate the interconnected interstitial space of the fibrous carrier structure with the reactive particles and bisphosphonate particles.

## Patentansprüche

1. Eine biokompatible, flexible, knochenadhäsive Folie, umfassend:
- eine kohäsive faserige Trägerstruktur, die einen dreidimensionalen, miteinander verbundenen Zwischenraum umfasst; und
- verteilt in dem Zwischenraum, (i) eine Vielzahl von Polymerteilchen, die ein wasserlösliches, kalziumbindendes Polymer umfassen, das aus Polyoxazolinen, Polyethylenglykolen, Polyvinylpyrrolidonen, Polyurethanen und Kombinationen davon ausgewählt ist, wobei das wasserlösliche, kalziumbindende Polymer mindestens eine kalziumbindende Gruppe trägt, die aus Bisphosphonat, Citrat, Ethylendiamintetraessigsäure (EDTA) und Kombinationen davon ausgewählt ist, oder (ii) eine Vielzahl von reaktiven Teilchen, die ein elektrophil aktiviertes wasserlösliches Polymer umfassen, das aus Polyoxazolinen, Polyethylenglykolen, Polyvinylpyrrolidonen, Polyurethanen und Kombinationen davon ausgewählt ist, und eine Vielzahl von Bisphosphonatteilchen, die stickstoffhaltiges Bisphosphonat umfassen,
wobei die calciumbindende Gruppe eine Gruppe ist, die in der Lage ist, zwei oder mehr separate koordinative Bindungen mit Ca2+-lonen zu bilden.

2. Knochenadhäsive Folie nach Anspruch 1, wobei die Fasern in der faserigen Trägerstruktur einen mittleren Durchmesser von 1-500 µm haben.

3. Knochenadhäsive Folie nach einem der vorhergehenden Ansprüche, wobei die faserige Trägerstruktur eine Filzstruktur, eine gewebte Struktur oder eine gestrickte Struktur, vorzugsweise eine Filzstruktur, aufweist.

4. Knochenadhäsive Folie nach einem der vorhergehenden Ansprüche, wobei die faserige Trägerstruktur mindestens 50 Gew.-% Fasern umfasst, die Gelatine, Kollagen, Cellulose, modifizierte Cellulose, Carboxymethyldextran, PLGA, Natriumhyaluronat/Carboxymethylcellulose, Polyvinylalkohol, Chitosan oder eine Kombination davon enthalten.

5. Knochenadhäsive Folie nach einem der vorhergehenden Ansprüche, wobei die knochenadhäsive Folie im Zwischenraum verteilt eine Vielzahl von Polymerteilchen umfasst, die ein wasserlösliches calciumbindendes Polymer umfassen, wobei das wasserlösliche calciumbindende Polymer mindestens eine calciumbindende Gruppe trägt.

6. Knochenadhäsive Folie nach Anspruch 5, wobei die faserige Trägerstruktur 5-90 % Polymerteilchen enthält, wobei der Prozentsatz mit dem Gewicht der faserigen Trägerstruktur berechnet wird.

7. Knochenadhäsive Folie nach Anspruch 5 oder 6, wobei die Polymerteilchen mindestens 10 Gew.-% des wasserlöslichen Calcium-bindenden Polymers enthalten.

8. Knochenadhäsive Folie nach einem der Ansprüche 5 bis 7, wobei die Polymerteilchen einen volumengewichteten mittleren Durchmesser im Bereich von 10 bis 100 µm aufweisen.

9. Knochenadhäsive Folie nach einem der Ansprüche 5 bis 8, wobei die kalziumbindenden Gruppen Bisphosphonat umfassen.

10. Knochenadhäsive Folie nach einem der Ansprüche 1 bis 4, wobei die knochenadhäsive Folie, in dem Zwischenraum verteilt, eine Vielzahl der reaktiven Teilchen umfasst, die ein elektrophil aktiviertes wasserlösliches Polymer umfassen, und eine Vielzahl der Bisphosphonatteilchen, die stickstoffhaltiges Bisphosphonat umfassen.

11. Knochenadhäsive Folie nach Anspruch 10, wobei die faserige Trägerstruktur 5-90 % der Kombination aus den reaktiven Teilchen und den Bisphosphonatteilchen enthält, wobei der Prozentsatz mit dem Gewicht der faserigen Trägerstruktur berechnet wird.

12. Knochenadhäsive Folie nach Anspruch 10 oder 11, wobei die reaktiven Teilchen und die Bisphosphonatteilchen in Agglomeraten kombiniert sind.

13. Knochenadhäsive Folie nach Anspruch 12, wobei die Agglomerate aus reaktiven Teilchen und Bisphosphonatteilchen einen volumengewichteten mittleren Durchmesser D [4,3] im Bereich von 100-400 µm aufweisen.

14. Knochenadhäsive Folie nach einem der Ansprüche 10 bis 13, wobei die reaktiven Teilchen mindestens 10 Gew.-% des elektrophil aktivierten wasserlöslichen Polymers enthalten.

15. Verfahren zur Herstellung einer knochenadhäsiven Folie nach einem der vorangehenden Ansprüche, wobei das Verfahren umfasst:
- Bereitstellung einer wasserbeständigen, kohäsiven faserigen Trägerstruktur, die einen dreidimensionalen, miteinander verbundenen Zwischenraum umfasst;
- Bereitstellen von Polymerteilchen, die ein wasserlösliches kalziumbindendes Polymer umfassen, wobei das wasserlösliche kalziumbindende Polymer mindestens eine kalziumbindende Gruppe trägt, oder Bereitstellen von reaktiven Teilchen, die ein elektrophil aktiviertes wasserlösliches Polymer umfassen, und Bisphosphonatteilchen, die stickstoffhaltiges Bisphosphonat umfassen;
- Imprägnieren der faserigen Trägerstruktur mit den Polymerpartikeln oder Imprägnieren der faserigen Trägerstruktur sowohl mit den reaktiven Partikeln als auch mit den Bisphosphonatteilchen.

16. Verfahren nach Anspruch 15, wobei die faserige Trägerstruktur imprägniert wird durch:
- Platzierung der faserigen Trägerstruktur und der Polymerteilchen zwischen zwei Elektroden;
- gleichzeitiges Aussetzen der faserigen Trägerstruktur und der Polymerteilchen einem elektrischen Feld von 0,1 bis 40 kV/mm, um den miteinander verbundenen Zwischenraum der faserigen Trägerstruktur mit den Polymerteilchen zu imprägnieren, oder gleichzeitiges Aussetzen der faserigen Trägerstruktur, der reaktiven Teilchen und der Bisphosphonatteilchen einem elektrischen Feld von 0,1 bis 40 kV/mm, um den miteinander verbundenen Zwischenraum der faserigen Trägerstruktur mit den reaktiven Teilchen und Bisphosphonatteilchen zu imprägnieren.

## Revendications

1. Feuille adhésive pour os flexible, biocompatible, comprenant :
une structure porteuse fibreuse cohésive comprenant un espace interstitiel interconnecté tridimensionnel ; et
réparties dans l'espace interstitiel, (i) une pluralité de particules de polymère comprenant un polymère de liaison au calcium hydrosoluble choisi parmi des polyoxazolines, des polyéthylène glycols, des polyvinylpyrrolidones, des polyuréthanes et des combinaisons de ceux-ci, ledit polymère de liaison au calcium hydrosoluble portant au moins un groupe de liaison au calcium choisi parmi un bisphosphonate, un citrate, un acide éthylènediaminetétraacétique (EDTA) et des combinaisons de ceux-ci ou (ii) une pluralité de particules réactives comprenant un polymère hydrosoluble activé électrophiliquement choisi parmi des polyoxazolines, des polyéthylène glycols, des polyvinylpyrrolidones, des polyuréthanes et des combinaisons de ceux-ci et une pluralité de particules de bisphosphonate comprenant du bisphosphonate azoté,
dans laquelle le groupe de liaison au calcium est un groupe qui est capable de former deux liaisons de coordination séparées ou plus avec des ions Ca²⁺.

2. Feuille adhésive pour os selon la revendication 1, dans laquelle les fibres dans la structure porteuse fibreuse présentent un diamètre moyen de 1 à 500 µm.

3. Feuille adhésive pour os selon l'une quelconque des revendications précédentes, dans lequel la structure porteuse fibreuse présente une structure de feutre, une structure tissée ou une structure tricotée, de préférence une structure de feutre.

4. Feuille adhésive pour os selon l'une quelconque des revendications précédentes, dans lequel la structure porteuse fibreuse comprend au moins 50 % en poids de fibres contenant de la gélatine, du collagène, de la cellulose, de la cellulose modifiée, du carboxyméthyldextrane, du PLGA, du hyaluronate de sodium/de la carboxyméthylcellulose, de l'alcool polyvinylique, du chitosane ou une combinaison de ceux-ci.

5. Feuille adhésive pour os selon l'une quelconque des revendications précédentes, dans laquelle la feuille adhésive pour os comprend, réparties dans l'espace interstitiel, une pluralité de particules de polymère comprenant un polymère de liaison au calcium hydrosoluble, ledit polymère de liaison au calcium hydrosoluble portant au moins un groupe de liaison au calcium.

6. Feuille adhésive pour os selon la revendication 5, dans laquelle la structure porteuse fibreuse contient de 5 à 90 % de particules de polymère, ledit pourcentage étant calculé en poids de la structure porteuse fibreuse.

7. Feuille adhésive pour os selon la revendication 5 ou 6, dans laquelle les particules de polymère contiennent au moins 10 % en poids de polymère de liaison au calcium hydrosoluble.

8. Feuille adhésive pour os selon l'une quelconque des revendications 5 à 7, dans lequel les particules de polymère présentent un diamètre moyen pondéré en volume dans la plage allant de 10 à 100 µm.

9. Feuille adhésive pour os selon l'une quelconque des revendications 5 à 8, dans laquelle les groupes de liaison au calcium comprennent du bisphosphonate.

10. Feuille adhésive pour os selon l'une quelconque des revendications 1 à 4, dans laquelle la feuille adhésive pour os comprend, réparties dans l'espace interstitiel, une pluralité des particules réactives comprenant un polymère hydrosoluble activé électrophiliquement et une pluralité des particules de bisphosphonate comprenant du bisphosphonate azoté.

11. Feuille adhésive pour os selon la revendication 10, dans laquelle la structure porteuse fibreuse contient de 5 à 90 % de la combinaison des particules réactives et des particules de bisphosphonate, ledit pourcentage étant calculé en poids de la structure porteuse fibreuse.

12. Feuille adhésive pour os selon la revendication 10 ou 11, dans laquelle les particules réactives et les particules de bisphosphonate sont combinées en agglomérats.

13. Feuille adhésive pour os selon la revendication 12, dans laquelle les agglomérats de particules réactives et de particules de bisphosphonate ont un diamètre moyen pondéré en volume D[4,3] dans la plage allant de 100 à 400 µm.

14. Feuille adhésive pour os selon l'une quelconque des revendications précédentes, dans laquelle les particules polymères réactives contiennent au moins 10 % en poids du polymère hydrosoluble activé électrophiliquement.

15. Procédé de préparation d'une feuille adhésive pour os selon l'une quelconque des revendications précédentes, ledit procédé comprenant les étapes consistant à :
fournir une structure porteuse fibreuse cohésive résistante à l'eau comprenant un espace interstitiel interconnecté tridimensionnel ;
fournir des particules polymères comprenant un polymère de liaison au calcium hydrosoluble, ledit polymère de liaison au calcium hydrosoluble portant au moins un groupe de liaison au calcium ou fournir des particules réactives comprenant un polymère hydrosoluble activé électrophiliquement et des particules de bisphosphonate comprenant du bisphosphonate azoté ;
imprégner la structure porteuse fibreuse avec les particules de polymère ou imprégner la structure porteuse fibreuse à la fois avec les particules réactives et les particules de bisphosphonate.

16. Procédé selon la revendication 15, dans lequel la structure porteuse fibreuse est imprégnée par les étapes consistant à :
mettre en place la structure porteuse fibreuse et les particules de polymère entre deux électrodes ;
soumettre simultanément la structure de support fibreuse et les particules de polymère à un champ électrique de 0,1 à 40 kV/mm pour imprégner l'espace interstitiel interconnecté de la structure porteuse fibreuse avec les particules de polymère, ou soumettre simultanément la structure porteuse fibreuse, les particules réactives et les particules de bisphosphonate à un champ électrique de 0,1 à 40 kV/mm pour imprégner l'espace interstitiel interconnecté de la structure porteuse fibreuse avec les particules réactives et les particules de bisphosphonate.
